# EUROPÄISCHE PATENTANMELDUNG

(11) **EP 3 808 336 A1**
(43) Veröffentlichungstag der Anmeldung: **21.04.2021**
(21) Anmeldenummer: 19203549.1
(22) Anmeldetag: 16.10.2019
(51) Int. Cl.: A61K 9/00, A61K 9/50, A61K 47/38, A61K 31/352

(54) **KONTROLLIERT FREISETZENDE FORMULIERUNGEN STARK LIPOPHILER PHYSIOLOGISCH AKTIVER SUBSTANZEN**

(71) Anmelder: ADD Advanced Drug Delivery Technologies, Ltd., 4133 Pratteln (CH)
(72) Erfinder: Nowak, Mirko, 79540 Lörrach (DE); Nowak, Jesko Jay, 79540 Lörrach (DE); Grave, Annette, 79541 Lörrach (DE); Wentzlaff, Monika, 79540 Lörrach (DE); Barthold, Sarah, 88444 Ummendorf (DE); Geugelin, Christian, 79588 Efringen-Kirchen (DE)
(74) Vertreter: Breuer, Markus

(57) **Zusammenfassung**

Es wird ein Produkt zur Freisetzung von stark lipophilen physiologisch aktiven Substanzen bereitgestellt, umfassend einen Kern und einen Überzug auf dem Kern, wobei der Überzug eine oder mehrere stark lipophile physiologisch aktive Substanzen, einen oder mehrere wasserlösliche Filmbildner und nicht mehr als 20 Gew.-%, bezogen auf das Gewicht aller Komponenten, an weiteren Hilfsstoffen umfasst.

## Beschreibung

### Gebiet der Erfindung

Die Erfindung betrifft Formulierungen für stark lipophile physiologisch aktive Substanzen, insbesondere kontrolliert freisetzende Formulierungen, sowie deren Herstellung. Bei den stark lipophilen physiologisch aktiven Substanzen handelt es sich beispielsweise um pharmazeutische Wirkstoffe. Ein Beispiel für stark lipophile pharmazeutische Wirkstoffe sind Cannabinoide.

### Hintergrund der Erfindung

Eine Reihe von physiologisch aktiven Substanzen haben stark lipophile Eigenschaften, d.h., sie weisen einen relativ hohen log P auf, beispielsweise einen log P von 4 oder mehr auf, wobei der log P der dekadische Logarithmus des n-Octanol/Wasser-Verteilungskoeffizienten ist.

Die Bereitstellung von Formulierungen, insbesondere oralen Formulierungen, mit solchen physiologisch aktiven Substanzen stellt eine besondere Herausforderung dar, insbesondere wenn eine kontrollierte Freisetzung der physiologisch aktiven Substanzen erzielt werden soll.

Zu den physiologisch aktiven Substanzen mit stark lipophilen Eigenschaften gehören Cannabinoide.

Cannabinoide sind eine heterogene Gruppe pharmakologisch aktiver Substanzen, die eine Affinität zu den sogenannten Cannabinoid-Rezeptoren aufweisen. Zu den Cannabinoiden gehören beispielsweise Tetrahydrocannabinol (THC) und das nicht psychoaktive Cannabidiol (CBD).

Cannabinoide haben erhebliches Interesse als Arzneistoffe gefunden. So gibt es Hinweise darauf, dass die Verabreichung von Cannabinoiden bei einer Reihe von klinischen Zuständen von Vorteil sein kann, darunter Schmerzen, Entzündungen, Epilepsie, Schlafstörungen, Symptome von Multipler Sklerose, Anorexie und Schizophrenie (N. Bruni et al., Cannabinoid Delivery Systems for Pain and Inflammation Treatment. Molecules 2018, 23, 2478).

Die Bereitstellung geeigneter Darreichungsformen ist allerdings mit Schwierigkeiten verbunden, da Cannabinoide stark lipophile Moleküle (log P 6-7) mit sehr geringer Wasserlöslichkeit (2-10 µg / ml) sind.

So hat die geringe orale Bioverfügbarkeit von Cannabinoiden dazu geführt, dass transdermale, intranasale und transmukosale Verabreichung vorgeschlagen wurden.

Daneben gehören aufgrund der hohen Lipophilie von Cannabinoiden Salzbildung (d.h. pH-Einstellung), Cosolvenz (z. B. Ethanol, Propylenglykol, PEG400), Mizellisierung (z. B. Polysorbat 80, Cremophor-ELP), Emulgierung einschließlich Mikro- und Nano-Emulgierung, Komplexierung (z. B. Cyclodextrine) und Einkapselung in Formulierungen auf Lipidbasis (z. B. Liposomen) zu den Formulierungsstrategien, die im Stand der Technik in Betracht gezogen wurden. Zudem wurden Nanopartikel-Systeme vorgeschlagen (N. Bruni et al., a.a.O.).

In der Patentliteratur sind verschiedene feste orale Darreichungsformen vorgeschlagen worden, so in WO 2008/024490 A2 und in WO 2018/035030 A1. Diese Dokumente enthalten keine Daten zum Freisetzungsverhalten, so dass die praktische Eignung der vorgeschlagenen Formen für die Verabreichung von Cannabinoiden unklar bleibt.

WO 2015/065179 A1 beschreibt komprimierte Tabletten, die neben Cannabidiol Lactose und Saccharosefettsäuremonoester enthalten.

Dronabinol (Δ9-THC) wird in Form von Kapseln (Marinol®) und als orale Lösung (Syndros®) vermarktet. Bei den Marinol®-Kapseln handelt es sich um Weichgelatinekapseln, die den Wirkstoff in Sesamöl enthalten.

Das Nabiximols-haltige Fertigarzneimittel Sativex® ist ein Mundspray, das auf die Innenseite der Wange gesprüht wird.

Das unlängst zugelassene Präparat Epidiolex zur Behandlung bestimmter Formen der Epilepsie wird in Form einer oralen Lösung zur Verfügung gestellt, die neben dem Wirkstoff Cannabidiol die Hilfsstoffe absolutes Ethanol, Sesamöl, Erdbeeraroma und Sucralose enthält.

Ungeachtet all dieser Vorschläge besteht aber weiterhin ein Bedarf an verbesserten Darreichungsformen für stark lipophile physiologisch aktive Substanzen, beispielsweise pharmazeutische Wirkstoffe wie Cannabinoide, insbesondere an oralen festen Darreichungsformen.

### Aufgaben und Kurzdarstellung der Erfindung

Eine Aufgabe der Erfindung besteht darin, feste Darreichungsformen, insbesondere orale feste Darreichungsformen, für stark lipophile physiologisch aktive Substanzen, wie Cannabinoide, bereitzustellen, die die physiologisch aktive Substanz/die physiologisch aktiven Substanzen freisetzen und die in einfacher Weise hergestellt werden können.

Diese Aufgabe wird durch die Bereitstellung eines Produkts zur Freisetzung von stark lipophilen physiologisch aktiven Substanzen gelöst, das einen Kern und einen Überzug auf dem Kern umfasst, wobei der Überzug eine oder mehrere stark lipophile physiologisch aktive Substanzen, einen oder mehrere wasserlösliche Filmbildner und nicht mehr als 20 Gew.-%, bezogen auf das Gewicht aller Komponenten, an weiteren Hilfsstoffen umfasst.

Überraschend wurde festgestellt, dass sich so feste Darreichungsformen, insbesondere orale feste Darreichungsformen, von stark lipophilen physiologisch aktiven Substanzen bereitstellen lassen, wobei über die Menge an Filmbildner(n), bezogen auf die Menge an stark lipophiler physiologisch aktiver Substanz/stark lipophilen physiologisch aktiven Substanzen, die Freisetzung gesteuert werden kann. Der Einsatz eines oder mehrerer Filmbildner erlaubt nicht nur die Ausbildung eines Überzugs mit der physiologisch aktiven Substanz/den physiologisch aktiven Substanzen, sondern dient auch der Steuerung der Freisetzung. Insbesondere fördert ein Filmbildner die Freisetzung der nur sehr wenig in Wasser löslichen stark lipophilen Substanzen. Erst durch den Filmbildner werden diese in ausreichender Menge und Geschwindigkeit freigesetzt.

Weitere Aufgaben und deren Lösung ergeben sich aus der nachstehenden ausführlichen Darstellung der Erfindung.

### Kurze Beschreibung der Figuren

Die Erfindung wird nachstehend unter Bezugnahme auf die Figur näher erläutert.

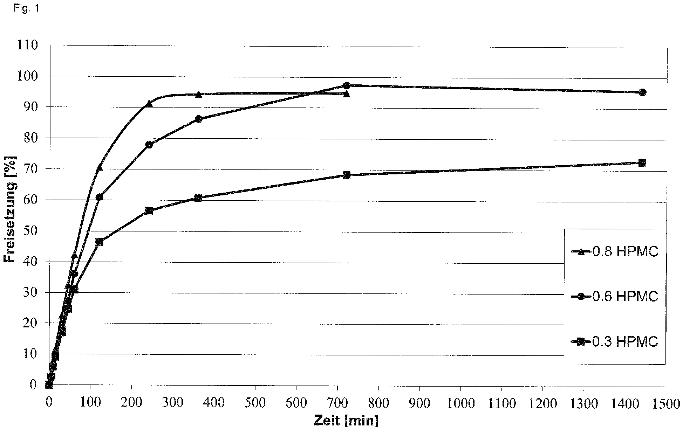

Fig. 1 zeigt die in vitro-Freisetzung aus drei Pelletprodukten, die 2-[1R-3-Methyl-6R-(1-methylethenyl)-2-cyclohexen-1-yl]-5-pentyl-1,3-benzenediol als Wirkstoff und niedrigviskose Hydroxypropylmethylcellulose als Filmbildner enthalten.

### Ausführliche Darstellung der Erfindung

Das erfindungsgemäß bereitgestellte Produkt enthält eine oder mehrere stark lipophile physiologisch aktive Substanzen.

Eine Substanz ist stark lipophil, wenn sie einen log P von 4 oder mehr hat. Der log P ist der dekadische Logarithmus des n-Octanol/Wasser-Verteilungskoeffizienten. Der Verteilungskoeffizient kann experimentell ermittelt werden. Werte beziehen sich typischerweise auf Raumtemperatur (25°C). Der Verteilungskoeffizient kann auch näherungsweise aus der Molekülstruktur errechnet werden.

Das erfindungsgemäße Produkt eignet sich besonders für physiologisch aktive Substanzen mit einem log P von 5 oder mehr und ganz besonders für solche mit einem log P von 6 oder mehr.

Der Begriff "physiologisch aktive Substanz" bezieht sich auf eine Substanz, die einem Menschen oder einem Tier verabreicht wird, um eine Wirkung im menschlichen oder tierischen Körper zu entfalten. Die physiologisch aktive Substanz kann beispielsweise ein pharmazeutischer Wirkstoff eines Human- oder Tierarzneimittels oder ein Nahrungsergänzungsmittel sein.

Ein Beispiel für stark lipophile pharmazeutische Wirkstoffe, die erfindungsgemäß verwendet werden können, sind Cannabinoide.

Bei den Cannabinoiden kann es sich sowohl um Phytocannabinoide als auch um synthetische Cannabinoide handeln.

Die Phytocannabinoide sind eine Gruppe von etwa 70 terpenophenolischen Verbindungen (V.R. Preedy (Hrsg.), Handbook of Cannabis and Related Pathologies (1997)). Diese Verbindungen enthalten typischerweise einen Monoterpenrest, der an einen Phenolring gebunden ist und eine C₃-C₅-Alkylkette aufweist, die sich in der meta-Position zur phenolischen Hydroxylgruppe befindet.

Eine bevorzugte Gruppe von Cannabinoiden sind Tetrahydrocannabinole der folgenden allgemeinen Formel (1): 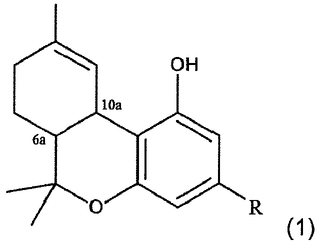 worin R ausgewählt ist aus C₁-C₂₀-Alkyl, C₂-C₂₀-Alkenyl oder C₂-C₂₀-Alkinyl und optional einen oder mehrere Substituenten aufweist.

In einer weiter bevorzugten Gruppe von Verbindungen der vorstehenden allgemeinen Formel (1) ist R ausgewählt aus C₁-C₁₀-Alkyl oder C₂-C₁₀-Alkenyl und weist optional einen oder mehrere Substituenten auf.

Insbesondere ist R in der Formel (1) ein Alkylrest der Formel C₅H₁₁.

Verbindungen der allgemeinen Formel (1) können in Form von Stereoisomeren vorliegen. Vorzugsweise weisen die Zentren 6a und 10a jeweils die R-Konfiguration auf.

Bei dem Tetrahydrocannabinol handelt es sich insbesondere um Δ9-THC mit dem chemischen Namen (6aR,10aR)-6,6,9-Trimethyl-3-pentyl-6a,7,8,10a-tetrahydro-6H-benzo[c]chromen-1-ol. Die Struktur wird durch die nachstehende Formel (2) wiedergegeben:

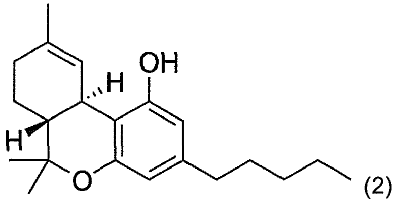

Eine weitere bevorzugte Gruppe von Cannabinoiden sind Cannabidiole der folgenden allgemeinen Formel (3): 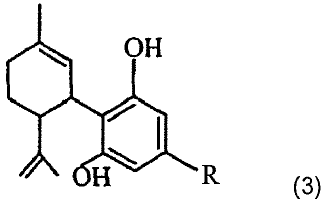 worin R ausgewählt ist aus C₁-C₂₀-Alkyl, C₂-C₂₀-Alkenyl oder C₂-C₂₀-Alkinyl und optional einen oder mehrere Substituenten aufweist.

In einer weiter bevorzugten Gruppe von Verbindungen der vorstehenden allgemeinen Formel (3) ist R ausgewählt aus C₁-C₁₀-Alkyl oder C₂-C₁₀-Alkenyl und weist optional einen oder mehrere Substituenten auf.

Insbesondere ist R in der Formel (3) ein Alkylrest der Formel C₅H₁₁.

Bei dem Cannabidiol handelt es sich insbesondere um 2-[1R-3-Methyl-6R-(1-methylethenyl)-2-cyclohexen-1-yl]-5-pentyl-1,3-benzenediol.

Erfindungsgemäß kann als Wirkstoff auch eine Kombination aus um Δ9-THC ((6aR,10aR)-6,6,9-Trimethyl-3-pentyl-6a,7,8,10a-tetrahydro-6H-benzo[c]chromen-1-οl) und CBD (2-[1R-3-Methyl-6R-(1-methylethenyl)-2-cyclohexen-1-yl]-5-pentyl-1,3-benzenediol) verwendet werden.

Eine weitere bevorzugte Gruppe von Cannabinoiden sind Cannabinole der folgenden allgemeinen Formel (4): 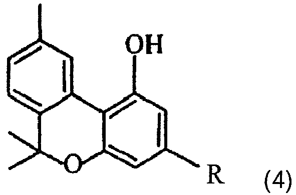 worin R ausgewählt ist aus C₁-C₂₀-Alkyl, C₂-C₂₀-Alkenyl oder C₂-C₂₀-Alkinyl und optional einen oder mehrere Substituenten aufweist.

In einer weiter bevorzugten Gruppe von Verbindungen der vorstehenden allgemeinen Formel (4) ist R ausgewählt aus C₁-C₁₀-Alkyl oder C₂-C₁₀-Alkenyl und weist optional einen oder mehrere Substituenten auf.

Insbesondere ist R in der Formel (4) ein Alkylrest der Formel C₅H₁₁.

Bei dem Cannabinol handelt es sich insbesondere um 6,6,9-Trimethyl-3-pentyl-6H-dibenzo[*b*,*d*]pyran-1-ol.

Erfindungsgemäß können auch Cannabinoide oder Cannabinoid-Gemische aus Hanfextrakten verwendet werden.

Beispielsweise ist Nabiximols eine als Arzneistoff verwendete Pflanzenextraktmischung aus den Blättern und Blüten der Hanfpflanze (Cannabis sativa L.) mit standardisierten Gehalten an Tetrahydrocannabinol (THC) und Cannabidiol (CBD).

Es können auch synthetische Cannabinoide verwendet werden.

Dazu gehört 3-(1,1-Dimethylheptyl)-6,6a,7,8,10,10a-hexahydro-1-hydroxy-6,6-dimethyl-9*H*-dibenzo[*b*,*d*]pyran-9-on. Diese Verbindung enthält zwei stereogene Zentren. Der Arzneistoff Nabilon ist ein 1:1-Gemisch (Racemat) aus der (6a*R*,10a*R*)-Form und der (6aS,10aS)-Form. Nabilon ist ein erfindungsgemäß bevorzugtes Cannabinoid.

Ein weiteres Beispiel für ein synthetisches Cannabinoid ist JWH-018 (1-Naphthyl-(1-pentylindol-3-yl)methanon.

Eine oder mehrere stark lipophile physiologisch aktive Substanzen, wie ein oder mehrere pharmazeutische Wirkstoffe, wie Cannabinoide, sind erfindungsgemäß in einem Überzug auf einem Kern enthalten. Dazu wird ein Kern mit einem Überzug versehen, der neben einer oder mehreren stark lipophilen aktiven Substanzen einen oder mehrere wasserlösliche Filmbildner umfasst. Neben der oder den stark lipophilen physiologisch aktiven Substanzen enthält der Überzug vorzugsweise keine weiteren physiologisch aktiven Substanzen.

Beispiele für geeignete wasserlösliche Filmbildner sind Methylcellulose (MC), Hydroxypropylmethylcellulose (HPMC), Hydroxypropylcellulose (HPC), Hydroxyethylcellulose (HEC), Natrium-Carboxymethylcellulose (Na-CMC) und Polyvinylpyrrolidon (PVP).

Bevorzugt ist Hydroxypropylmethylcellulose (HPMC), insbesondere niedrigviskose HPMC, wie HPMC mit einer Viskosität einer 2% (Gew./Gew.) wässrigen Lösung bei 20°C von 6 mPa·s oder weniger.

Eine HPMC mit einer Viskosität einer 2% (Gew./Gew.) wässrigen Lösung bei 20°C von 3 mPa·s, wie sie unter der Handelsbezeichnung Pharmacoat® 603 verfügbar ist, ist besonders bevorzugt.

Der Überzug aus einer oder mehreren stark lipophilen physiologisch aktiven Substanzen und einem oder mehreren wasserlöslichen Filmbildnern kann weitere übliche Hilfsstoffe enthalten. Die Menge der weiteren Hilfsstoffe ist erfindungsgemäß auf nicht mehr als 20 Gew.-%, bezogen auf das Gewicht aller Komponenten, begrenzt. Vorzugsweise sind nicht mehr als 10 Gew.-%, bezogen auf das Gewicht aller Komponenten, an weiteren Hilfsstoffen enthalten.

In einer besonders bevorzugten Ausführungsform besteht der Überzug aus stark lipophiler physiologisch aktiver Substanz/stark lipophilen physiologisch aktiven Substanzen und Filmbildner(n), beispielsweise aus Cannabinoid(en) und Filmbildner(n).

Erfindungsgemäße Pellets weisen einen Überzug auf, der einen oder mehrere wasserlösliche Filmbildner, bezogen auf die Gesamtmenge an stark lipophilen physiologisch aktiven Substanzen, in einem Gesamtanteil von 0,1 - 10 Gew.-%, vorzugsweise in einem Gesamtanteil von 0,5 - 8 Gew.-% und insbesondere in einem Gesamtanteil von 1-6 Gew.-% enthält.

Es wird angenommen, dass bei einer zu geringen Menge an Filmbildner die Freisetzung nur sehr langsam und unvollständig erfolgt. Durch Wahl des Anteils in den angegebenen Bereichen kann die Freisetzung der physiologisch aktiven Substanz eingestellt werden. Beispielsweise kann die Freisetzung aus einer oralen Darreichungsform so eingestellt werden, dass die physiologisch aktive Substanz über die übliche Zeit der Magen-Darm-Passage abgegeben wird.

Der Überzug wird auf Kerne aufgebracht. Die Kerne können beliebig aufgebaut sein und aus beliebigen, physiologisch annehmbaren Materialien bestehen. Als Kerne können beispielsweise Tabletten, Minitabletten, Pellets, Granulatkörner oder Kristalle verwendet werden. Die Kerne können beispielsweise Zucker, Weinsäure oder mikrokristalline Cellulose enthalten oder daraus bestehen. Bevorzugt sind inerte Starterkerne, wie Pellets aus mikrokristalliner Cellulose. Solche Pellets sind unter der Bezeichnung Cellets® im Handel erhältlich.

Die Größe der Kerne ist nicht beschränkt. Geeignete Größen liegen im Bereich von 10 µm bis 2000 µm, beispielsweise im Bereich von 50 µm bis 1500 µm und vorzugsweise 100 µm bis 1000 µm liegen, wobei die Größe durch Siebanalyse bestimmt werden kann. Insbesondere können Pellets aus einer Siebfraktion von 500 - 710 µm verwendet werden.

Die erfindungsgemäßen Produkte können hergestellt werden, indem zunächst eine Sprühflüssigkeit hergestellt wird, die eine oder mehrere stark lipophile physiologisch aktive Substanzen und einen oder mehrere wasserlösliche Filmbildner enthält.

Da stark lipophile Substanzen nur eine sehr geringe Wasserlöslichkeit aufweisen, kommt typischerweise ein organisches Lösungsmittel oder ein Gemisch aus einem organischen Lösungsmittel und Wasser zum Einsatz.

Die Sprühflüssigkeit wird dann auf Kerne aufgebracht. Dabei werden die flüssigen Anteile verdampft, so dass ein Überzug auf den Kernen gebildet wird, der weitgehend frei von Lösungsmittel und Wasser ist. Dies kann beispielsweise in einer Wirbelschichtanlage, einer Strahlschichtanlage, einem Sprühtrockner oder einem Coater erfolgen.

Überzogene Kerne können anschließend als orale Darreichungsform verwendet werden. Überzogene Pellets können z.B. in Sachets angeboten werden, oder sie können weiterverarbeitet werden.

Die erfindungsgemäß überzogenen Kerne können auch mit einem oder mehreren weiteren Überzügen versehen werden. Damit ist eine zusätzliche Steuerung der Freisetzung möglich.

In einer bevorzugten Ausführungsform ist kein weiterer Überzug vorgesehen, der die Freisetzung steuert.

Überzogene Pellets können auch verwendet werden, um multipartikuläre Darreichungsformen zu erhalten. Sie können beispielsweise in Kapseln gefüllt oder in Tabletten eingearbeitet werden. Gemäß einer Ausführungsform werden sie zu oral dispersible tablets verarbeitet.

Überzogene Pellets mit unterschiedlichen Freisetzungsprofilen können in einer Darreichungsform (Kapsel/Tablette/Sachet) kombiniert werden. Die erfindungsgemäßen Produkte setzen die enthaltene stark lipophile physiologisch aktive Substanz oder, falls mehr als eine stark lipophile physiologisch aktive Substanz enthalten ist, sämtliche enthaltenen stark lipophilen physiologisch aktiven Substanzen nach Einnahme im Verdauungstrakt frei. Die Produkte dienen insbesondere der kontrollierten Freisetzung. Insbesondere setzen sie mehr als 30 Gew.-% und weniger als 80 Gew.-% der enthaltenen physiologisch aktiven Substanz innerhalb von zwei Stunden frei. Weiterhin setzen sie insbesondere mehr als 40 Gew.-% und weniger als 90 Gew.-% der enthaltenen physiologisch aktiven Substanz innerhalb von drei Stunden frei. Darüber hinaus setzen sie mehr als 50 Gew.-% und weniger als 95 Gew.-% der enthaltenen physiologisch aktiven Substanz innerhalb von vier Stunden frei. Falls mehr als eine physiologisch aktive Substanz enthalten ist, beziehen sich die Angaben auf sämtliche enthaltenen Substanzen.

Die Bestimmung der Freisetzung erfolgt dabei jeweils in einer Blattrührer-Apparatur in 1000 ml Phosphatpuffer pH 6.8 mit einem Zusatz an 0.4 % Tween® 80 bei 37° C.

### Beispiele

Die Erfindung wird anhand konkreter Anwendungsbeispiele veranschaulicht, ohne dadurch in irgendeiner Weise eingeschränkt zu werden.

### Beispiel 1 - Herstellung von Pellets

Es wurden Pellets unter Verwendung der in der nachstehenden Tabelle 1 angegebenen Mengen an Inhaltsstoffen hergestellt.

Dazu wurde 2-[1R-3-Methyl-6R-(1-methylethenyl)-2-cyclohexen-1-yl]-5-pentyl-1,3-benzenediol (Canapure PH) in Ethanol 96% gelöst. Dieser Wirkstoff weist einen log P von etwa 6,1 auf.

Eine weitere Lösung wurde hergestellt, indem HPMC (Pharmacoat® 603) in Wasser gelöst wurde.

Anschließend wurde die HPMC-Lösung schrittweise zu der Cannabidiol-Lösung gegeben.

Dann wurde amorphes Siliciumdioxid (Syloid® 244 FP) zugefügt.

Es wurde jeweils mit einem Propellerrüher gerührt.

Die erhaltene Sprühflüssigkeit wurde auf Starterkerne aus mikrokristalliner Cellulose (Cellets® 500) aufgesprüht.

Dies erfolgte in einer Wirbelschichtanlage vom Typ Mini-Glatt mit einem Wurster-Einsatz. Dabei betrug die Lufteinlasslufttemperatur 40°C. Die mittlere Sprührate betrug 0,5 g/min.

**Tabelle 1 - Eingesetzte Stoffe und Mengen**

| **Ansatz** | **HPMC 0.8** | **HPMC 0.6** | **HPMC 0.3** |
|---|---|---|---|
| **Feststoffe** | Menge | Menge | Menge |
| Cellets 500 | 60,01 g / 81,5 % | 60,00 g / 72,7 % | 60,00 g / 72,7 % |
| Canapure PH | 21,02 g / 16,1 % | 21,00 g / 24,2 % | 21,26 g / 24,5 % |
| Pharmacoat 603 | 1,05 g / 0,8 % | 0,53 g / 0,6 % | 0,26 g / 0,3 % |
| Syloid 244 FP | 2,10 g / 1,6 % | 2,10 g / 2,4 % | 2,10 g / 2,4 % |

| **Flüssigkeiten (nicht im Produkt enthalten)** | | | |
|---|---|---|---|
| Ethanol 96% | 79,81 g | 79,83 g | 79,82 g |
| Reinwasser | 25,20 g | 25,21 g | 25,21 g |

| **Sprühflüssigkeit** | | | |
|---|---|---|---|
| Feststoffanteil (Gew./Gew.) | 18,71 % | 18,36 % | 18,36 % |
| Versprühte Menge | 72,80 g | 122,50 g | 122,50 g |

**Tabelle 2 - Produkte**

| **Ansatz** | **HPMC 0.8** | **HPMC 0.6** | **HPMC 0.3** |
|---|---|---|---|
| Theoretische Ausbeute | 73,63 g | 82,49 g | 82,49 g |
| Praktische Ausbeute | 64,30 g / 87,33 % | 75,03 g / 90,95 % | 74,24 g / 90,00 % |
| Überzugsgewichtszunahme | 31,49 % | 66,82 % | 63,31 % |

### Beispiel 2 - Freisetzung

Die Freisetzung aus den in Beispiel 1 erhaltenen Pelletprodukten wird unter Verwendung einer Blattrührer-Apparatur in 1000 ml Phosphatpuffer pH 6.8 mit einem Zusatz an 0.4 % Tween® 80 untersucht, und zwar bei 37° C. Die erhaltenen Ergebnisse sind in Fig. 1 gezeigt.

## Patentansprüche

1. Produkt zur Freisetzung von stark lipophilen physiologisch aktiven Substanzen, umfassend einen Kern und einen Überzug auf dem Kern, wobei der Überzug eine oder mehrere stark lipophile physiologisch aktive Substanzen, einen oder mehrere wasserlösliche Filmbildner und nicht mehr als 20 Gew.-%, bezogen auf das Gewicht aller Komponenten, an weiteren Hilfsstoffen umfasst, wobei eine physiologisch aktive Substanz stark lipophil ist, wenn sie einen log P von 4 oder mehr hat.

2. Produkt nach Anspruch 1, wobei als stark lipophile physiologisch aktive Substanzen ein oder mehrere stark lipophile pharmazeutische Wirkstoffe enthalten sind.

3. Produkt nach Anspruch 2, wobei als stark lipophile Wirkstoffe ein oder mehrere Cannabinoide enthalten sind.

4. Produkt nach Anspruch 3, wobei ein oder mehrere Cannabinoide enthalten sind, die ausgewählt sind aus Verbindungen einer der folgenden allgemeinen Formeln (1), (3) oder (4): 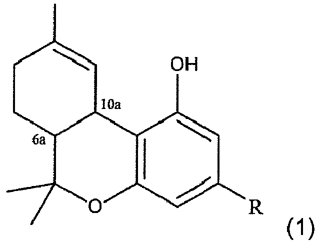 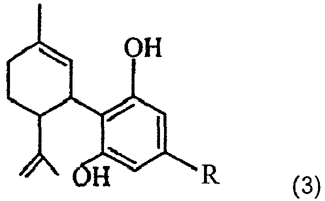 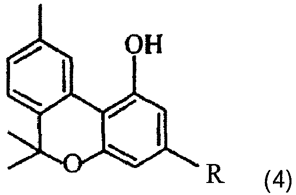
worin R jeweils ausgewählt ist aus C₁-C₂₀-Alkyl, C₂-C₂₀-Alkenyl oder C₂-C₂₀-Alkinyl und optional einen oder mehrere Substituenten aufweist;
Cannabinoiden oder Cannabinoid-Gemischen aus Hanfextrakten, wie Nabiximols; und
synthetischen Cannabinoiden, wie Nabilon oder 1-Naphthyl-(1-pentylindol-3-yl)methanon.

5. Produkt nach Anspruch 4, wobei es sich bei dem Cannabinoid um 2-[1R-3-Methyl-6R-(1-methylethenyl)-2-cyclohexen-1-yl]-5-pentyl-1,3-benzenediol; (6aR,10aR)-6,6,9-Trimethyl-3-pentyl-6a,7,8,10a-tetrahydro-6H-benzo[c]chromen-1-ol oder ein Gemisch davon handelt.

6. Produkt nach einem der vorstehenden Ansprüche, wobei als wasserlöslicher Filmbildner Hydroxypropylmethylcellulose (HPMC) eingesetzt wird.

7. Produkt nach Anspruch 6, wobei die HPMC eine Viskosität von 6 mPa·s oder weniger in einer 2% (Gew./Gew.) wässrigen Lösung bei 20°C aufweist.

8. Produkt nach einem der vorstehenden Ansprüche, wobei der Filmbildner/die Filmbildner, bezogen auf die Gesamtmenge an stark lipophilen aktiven Substanzen, in einem Gesamtanteil von 0,3 - 10 Gew.-%, vorzugsweise in einem Gesamtanteil von 0,5 - 8 Gew.-% und insbesondere in einem Gesamtanteil von 1 - 6 Gew.-%, enthalten ist/sind.

9. Produkt nach einem der vorstehenden Ansprüche, wobei der Überzug auf inerte Starterkerne aufgebracht ist.

10. Produkt nach Anspruch 9, wobei die Starterkerne eine Größe im Bereich von 10 µm bis 2000 µm, beispielsweise im Bereich von 50 µm bis 1500 µm und vorzugsweise 100 µm bis 1000 µm haben, wobei die Größe durch Siebanalyse bestimmt wird.

11. Produkt nach Anspruch 9 oder 10, wobei als Starterkerne Neutralpellets aus mikrokristalliner Cellulose verwendet werden.

12. Produkt nach einem der vorstehenden Ansprüche, das mehr als 30 Gew.-% und weniger als 80 Gew.-% der enthaltenen physiologisch aktiven Substanz/der enthaltenen physiologisch aktiven Substanzen innerhalb von zwei Stunden freisetzt.

13. Produkt nach Anspruch 12, das mehr als 40 Gew.-% und weniger als 90 Gew.-% der enthaltenen physiologisch aktiven Substanz/der enthaltenen physiologisch aktiven Substanzen innerhalb von drei Stunden freisetzt.

14. Produkt nach Anspruch 13, das mehr als 50 Gew.-% und weniger als 95 Gew.-% der enthaltenen physiologisch aktiven Substanz/der enthaltenen physiologisch aktiven Substanzen innerhalb von vier Stunden freisetzt.

15. Multipartikuläre Darreichungsform auf der Basis eines Produkts nach einem der vorstehenden Ansprüche.
